(19) Europäisches Patentamt
European Patent Office
Office européen des brevets

(11) Publication number: 0 421 600 A1

(12) # EUROPEAN PATENT APPLICATION

(21) Application number: 90309479.5

(22) Date of filing: 30.08.90

(51) Int. Cl.5: C07C 49/80, C07C 33/46, C07D 303/08, C07D 309/12, C07D 407/12, C07D 249/08, C07D 405/12

(30) Priority: 01.09.89 US 402014

(43) Date of publication of application: 10.04.91 Bulletin 91/15

(84) Designated Contracting States: GR

(71) Applicant: SCHERING CORPORATION
2000 Galloping Hill Road
Kenilworth New Jersey 07033(US)

(72) Inventor: Girijavallabhan, Viyyoor M.
10 Maplewood Drive
Parsippany, New Jersey 07054(US)

Inventor: Pinto, Patrick
232 Randolph Avenue
Mine Hill, New Jersey 07801(US)
Inventor: Ganguly, Ashit K.
96 Cooper Avenue
Upper Montclair, New Jersexy 07043(US)
Inventor: Sarre, Olga Z.
12 Valhalla Way
Verona, New Jersey 07044(US)

(74) Representative: Ritter, Stephen David et al
Mathys & Squire 10 Fleet Street
London EC4Y 1AY(GB)

(54) Asymmetric chemical synthesis and intermediates for making antifungal compounds.

(57) Intermediates of the structural formula II, III, IV, VIII, XI, or XII, wherein said compound has an asymmetric center(s) as indicated by the single asterisk (*) and the double asterisk (**) and wherein the compound is substantially free from compound(s) having the same chemical structure but having other absolute stereochemical configurations at the asterisked carbon centers:

EP 0 421 600 A1

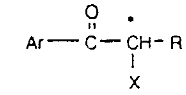 II,

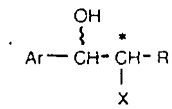 III,

1

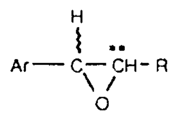 IV,

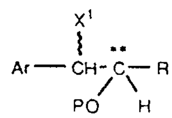 VIII,

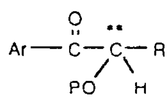 XI or

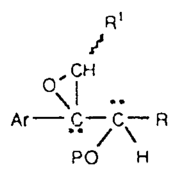 XII

or a pharmaceutically acceptable salt thereof,

wherein Ar represents phenyl, substituted phenyl, 3-thienyl, substituted 3- thienyl, 3-furanyl or substituted 3-furanyl; R represents alkyl or cycloalkyl; $R^1$ represents H or alkyl; X represents chloro, bromo or iodo; $X^1$ represents OH, chloro, bromo, iodo or $P^1$ where $P^1$ represents alkoxy-substituted phenyloxy, benzyloxy, substituted benzyloxy, acyloxy, alkylthio, phenylthio, substituted phenylthio or $-NHP^2$ where $P^2$ is an amino protecting group; and P represents H or a hydroxy protecting group; and processes for making chemically pure, enantiomeric antifungal compounds.

# ASYMMETRLC CHEMICAL SYNTHESIS AND INTERMEDIATESFOR MAKING ANTIFUNGAL COMPOUNDS

## BACKGROUND OF THE INVENTION

European Published Application Nos. 178533 and 61835 disclose certain antifungal compounds having two asymmetric centers of the formula:

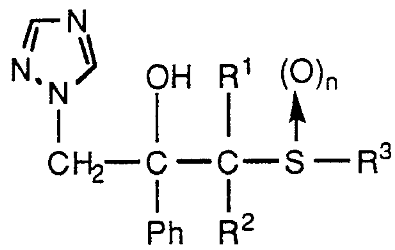

wherein Ph is a phenyl group or a phenyl group substituted with one or two halogen atoms, $R^1$ is a $C_1$-$C_3$ alkyl group, $R^2$ is a hydrogen atom or a $C_1$-$C_3$ alkyl group, $R^3$ is a $C_1$-$C_8$ alkyl group, a $C_4$-$C_8$ cycloalkylalkyl group or a $C_3$-$C_6$ cyclalkyl group and n is 0, 1 or 2, and their acid addition salts. Certain of these isomers have been found to provide more advantageous properties than the racemic mixture from which they are derived. Neither published application, however, discloses any asymmetric chemical syntheses for making the individual isomers. It would be highly advantageous to provide processes and intermediates by which the individual, pure isomers could be prepared economically and in good yield.

U.S. Patent No. 4,526,983 discloses a process for making certain optical active antifungal compounds having the formula:

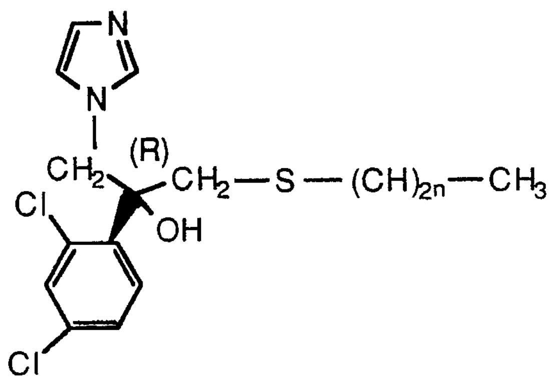

wherein n is an integer of 3 or 4, and its acid addition salts, by diastereomeric separation using an optically active carboxylic acid ester. This is not suitable for large scale commercial synthesis. It is laborious, low yielding and expensive.

The present invention involves a series of important novel intermediates useful in making chemically pure, enantiomeric compounds having antifungal activity. One aspect of the invention involves an intermediate compound of the formula II, III, IV, VIII, XI or XII, wherein said compound has an asymmetric center(s) as indicated by the single asterisk (*) and the double asterisk (**) and wherein the compound is substantially free from compound(s) having the same chemical structure but having other absolute stereochemical configurations at the asterisked carbon centers (e.g., the double asterisks (**) in formula XII both represent the R absolute stereochemical configurations, and such RR compound is substantially free from its SS, RS and SR forms):

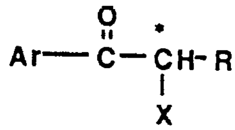 II,

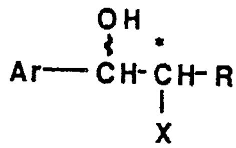 III,

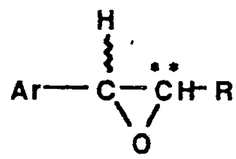 IV,

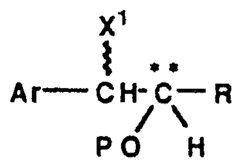 VIII,

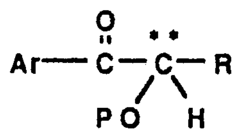 XI or

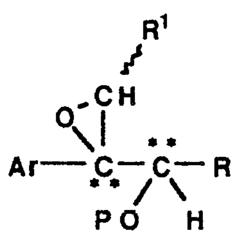 XII

or a pharmaceutically acceptable salt thereof, wherein Ar represents phenyl, substituted phenyl, 3-thienyl, substituted 3- thienyl, 3-furanyl or substituted 3-furanyl; R represents alkyl, substituted alkyl or cycloalkyl; $R^1$ represents H or alkyl; X represents chloro, bromo or iodo; $X^1$ represents OH, chloro, bromo, iodo or $P^1$ where $P^1$ represents alkoxy-substituted phenyloxy, benzyloxy, substituted benzyloxy, acyloxy, alkylthio, phenylthio, substituted phenylthio or -$NHP^2$ where $P^2$ is an amino protecting group such as phenyl, substituted phenyl, benzyl, substituted benzyl, alkanoyl, alkoxycarbonyl, phenoxycarbonyl or substituted phenoxycarbonyl; and P represents H or a hydroxy protecting group.

A preferred intermediate of the invention is of the formula XII, wherein Ar, R, $R^1$ and P are as defined above. Preferably, the double asterisked (**) carbon atoms in the compound of formula XII are both in their R absolute stereochemicai configuration, i.e., the RR form of the compound. However, it is noted that the SS form of formula XII also is within the scope of this invention.

Another preferred intermediate of the invention is of formula XI, wherein Ar, P and R are as defined above. In the preferred form of the compound of formula XI the carbon atom indicated by the double asterisk (**) is in its R absolute stereochemical configuration.

The asymmetric centers indicated by the single asterisk (*) in the compounds of formulas II and III are preferably in their S absolute stereochemical configuration, while the asymmetric centers indicated by the double asterisk (**) in the compounds of formulas IV and VIII are preferably in their R absolute stereochemical configuration.

In the above formulas, Ar is preferably substituted phenyl, more preferably Ar is mono, di or tri-substituted halo phenyl, e.g., 2,4-difluoro, 2,6-difluoro, 4-fluoro, 2-fluoro, 2,4-dichloro, 2,6-dichloro, 4-chloro or 2-chloro substituted phenyl. A particularly preferred Ar group is 2,4-difluorophenyl. R is preferably alkyl

and in particular methyl. P is preferably

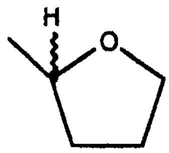
,
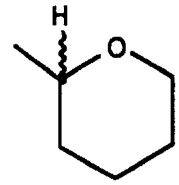
,
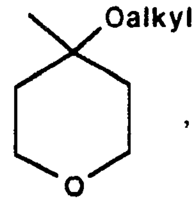
,

alkyloxyalkyl, alkylthioalkyl or $R'R''R'''$silyl where $R'$, $R''$ and $R'''$ may be the same or different and each is selected from alkyl, cycloalkyl or phenyl. More preferably, P is 2-tetrahydropyranyl. $R^1$ is preferably H. X is preferably chloro or bromo. $X^1$ is preferably chloro, bromo, alkoxy-substituted phenyloxy, benzyloxy or substituted benzyloxy.

The above novel intermediates provide very advantageous starting materials for making the desired antifungal end products having the formula XVI

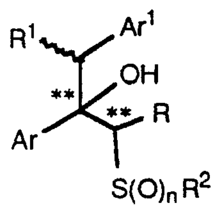
XVI,

having asymmetric carbon centers indicated by the asterisk (*), wherein both asterisked carbon centers have the same absolute stereochemical configuration, and wherein said compound is substantially free from compounds having other absolute stereochemical configurations at such carbon centers:

wherein Ar represents phenyl, substituted phenyl, 2- or 3-thienyl, substituted 2- or 3-thienyl, 2- or 3-furanyl, substituted 2- or 3-furanyl, 2- ,4- or 5-imidazolyl, 3- or 5-(1 ,2,4-triazolyl), 5-tetrazolyl, 2-, 4- or 5-thiazolyl, 2-, 4- or 5-oxazolyl, 3-, 4- or 5-isoxazolyl, 2-,3- or 4-pyridinyl, 2- or 3-pyrrolyl, 3- or 4-pyrrazolyl, 2-benzimidazolyl, 2-benzthiazolyl, or 2-, 4- or 6-purinyl, wherein the saturated nitrogen atoms on said imidazolyl, triazolyl, tetrazolyl, benzimidazolyl, benzthiazolyl, and purinyl groups may be substituted with H, alkyl, substituted alkyl, cycloalkyl, substituted cycloalkyl, phenyl or substituted phenyl;

$Ar^1$ represents 1 -(1 ,2,4-thiazolyl), 1 -(1 ,3,4-triazolyl) or 1-imidazolyl;

$R^2$ is alkyl, cycloalkyl or cycloalkylalkyl; and

wherein n is 1 or 2.

In the processes described below Ar, R, $R^1$, X, $X^1$, and P are as defined above unless otherwise indicated. The wary lines indicate mixtures of the possible isomers (as also indicated by R/S) and the asterisks (*) indicate (here and throughout this specification) an asymmetric carbon center in its absolute stereochemical R or S form, which is substantially free of its opposite enantiomeric form. In the processes described beow, the double asterick (**) represents an asymmetric carbon center which has the opposite absolute stereochemical configuration than a single asterisk (*) carbon in the reaction scheme. For example, if the asterisk (* )indicates an absolute stereochemical R form, the double asterisk (**) indicates an absolute stereochemical S form and vise versa.

Thus, one process aspect of the invention involves reacting a compound of the formula ArH with a compound of formula I substantially free from its opposite enantiomeric form:

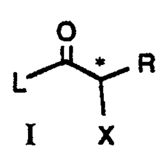
I

wherein the asterisk (*) indicates the asymmetric center (preferably in its S absolute stereochemical configuration) and wherein L represents a suitable leaving group such as halo, acid anhydride, etc., in the presence of a Lewis acid such as $AlCl_3$, $SnCl_4$, etc., to form a compound of formula II

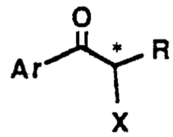 II;

reacting the compound of formula II with a non-basic, reducing agent to form a compound of the formula III

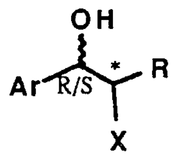 III;

and reacting the compound of the formula III with a base to form a compound of the formula IV

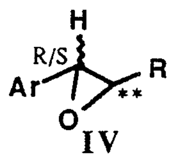
IV

wherein the asymmetric center indicated by the double asterisk (**) is inverted (preferably to the R form) with respect to the asymmetric centers indicated by the single asterisk (*) above. This process is advantagous in that it starts with generally commercially available or easily obtainable compounds of formula I. Surprisingly, in the reaction with ArH, no racemization is obtained. Also, enantiomeric integrity is retained in the reduction of the compound of formula II to the compound of the formula III using various reducing agents. Further, the process allows inversion at the X group carbon atom in the compound of formula I (with respect to the same carbon in formula IV) with about 100% specificity. Thus, in the above process when the carbon atom indicated by the asterisk (*) in formula I is in its S absolute stereochemical configuration, the same carbon atom in formula IV indicated by the double asterisk (**) will be in its R absolute stereochemical configuration and when the asterisk (*) in formula I represents the R absolute stereochemical configuration, the carbon atom the double asterisk (**) in formula IV will be in its S absolute stereochemical configuration. Likewise, in the subsequent reaction shown below the stereochemistry represented by the asterisk (*) and the double asterisk (**) has the same relationship.

In another aspect, the compound of formula IV

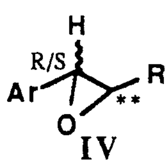
IV

wherein the double asterisk (**) indicates an asymmetric center (preferably in the R absolute stereochemical configuration), is reacted with an epoxide opening agent to form a compound of the formula V

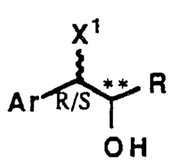 V

wherein the absolute stereochemical configuration at the double asterisk (**)asymmetric center is substantially retained. The compound of formula V may then be reacted with a hydroxy protecting agent to form a compound of the formula VIII

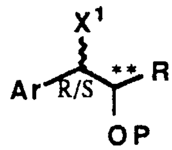
VIII.

The compound of formula VIII may then be reacted in one or two steps with an oxidizing agent to form a compound of the formula XI

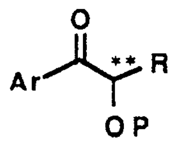
XI.

The compound of the formula VIII where $X^1$ is halo, OH or a hydroxy derived leaving group such as mesylate may also be reacted with a reducing agent to form a compound of the formula IX

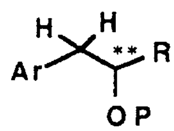
IX,

which in turn may be reacted with an oxidizing agent to form a compound of the formula XI

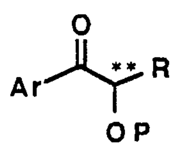
XI.

In another aspect of the invention, the compound of formula IV

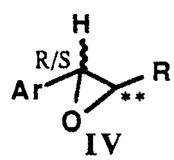
IV

wherein the double asterisk (**) indicates an asymmetric center (preferably in the R absolute stereochemical configuration), is reacted with an oxidizing agent (and a hydrolyzing agent such as water for the compound of formula VI) to form a compound of the formula VI and/or VII

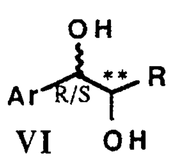
VI

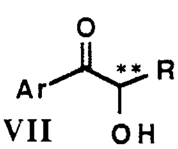
VII

wherein the absolute stereochemical configuration at the double asterisk (**) asymmetric center is substantially retained. The compound of formula VI or VII may then be reacted with a hydroxy protecting agent to form a compound of the formula X or XI, respectively:

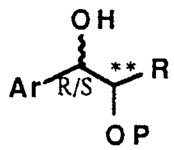 X or 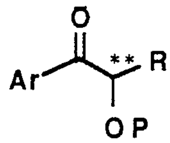 XI.

The compound of the formula X may be oxidized to the compound of the formula XI. The compound of formula XI may then be reacted with an epoxidizing agent, e.g., a dialkyl sulfoxide anion, to form a compound of the formula XII

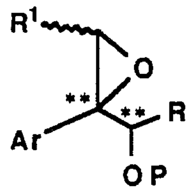 XII.

Thus, where dimethyl sulfoxide is employed, $R^1$ will be H. Surprisingly, the stereochemical configuration at the newly formed asymmetric center attached to Ar is substantially the same as the configuration at the center attached to R.

The compound of the formula XII may then be employed to prepare the desired antifungal end products of formula XVI by reaction with a nucleophilic $Ar^1$ anion and subsequent removal of any protecting group P to form a compound of the formula XIII

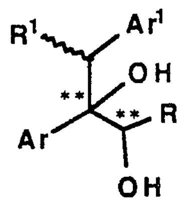 XIII

wherein $Ar^1$ represents 1-(1, 2, 4-triazolyl), 1-(1, 3, 4-triazolyl) or 1-imidazolyl. This intermediate compound of formula XIII is disclosed in racemic form in European published application No. 0178533.

The compound of formula XIII is then converted into the compound of formula XVI by applying methods known per se to compound of formula XIII such as those as described in the European publication i.e. for example, XIII is reacted with a methane sulfonating agent such as methane sulfonyl chloride to form a compound of the formula XIV

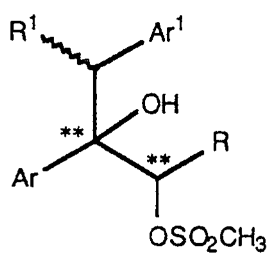 XIV;

which is then reacted with a mercaptan anion of the formula $SR^2$ to form a compound of formula XV

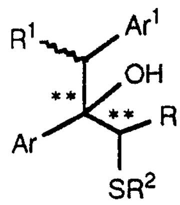

XV,

where $R^2$ is alkyl, cycloalkyl or cycloalkylalkyl, which in turn is reacted with an oxidizing agent to form a compound of the formula XVI

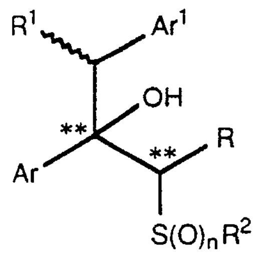

XVI

wherein n is 1 or 2. $Ar^1$ is preferably 1 -(1,2,4-triazolyl), Ar is preferably 2,4-diflourophenyl, R and $R^2$ are preferably methyl, $R^1$ is preferably H, n is preferably 2, and both of the carbon atoms indicated by the double asterisk (**) are in the R absolute stereochemical configuration. Using similar chemistry and starting with a compound of formula I having carbon the single asterisk (*) in an R absolute stereochemical configuration, compound XVI could be obtained possessing S absolute stereochemical configuration at both the double asterisked (**) asymmetric carbons.

This invention relates to substantially pure enantiomers and to processes for making such substantially pure enantiomers. By substantially pure, we mean substantially free from any of the other enantiomeric forms of such compounds, e.g., one enantiomer is present in amounts greater than about 95 molar %, more preferably greater than about 97 molar %, most preferably greater than about 99 molar %, with respect to any other enantiomer.

The following terms when used herein have the meanings given below, unless otherwise indicated:

acyl - represents alkanoyl, halo-substituted alkyl carbonyl, benzoyl and substituted benzoyl;

alkyl (including the alkyl portions of alkylthio, alkoxy,

alkyloxyalkyl, alklythioalkyl, alkanoyl, alkoxycarbonyl, hydroxyalkyl, etc.) - represents a straight or branched hydrocarbon chain having from 1 to 12, preferably from 1 to 6, carbon atoms;

substituted alkyl - represents an alkyl group as defined above wherein 1 to 3 of the hydrogen atoms are replaced with halo, protected hydroxy, phenyl, substituted phenyl which is not reactive with a Lewis acid, etc.;

cycloalkyl (including the cycloalkyl portion of cycloalkylalkyl) - represents a saturated carbocyclic ring having from 3 to 8, preferably from 3 to 6, carbon atoms;

halo - represents fluoro, chloro, bromo or iodo;

amino protecting group - represents a group which will protect an amino group during a desired chemical reaction such as the reactions described above, e.g., suitable amino protecting groups include benzyl, substituted benzyl, alkoxy-substituted phenyl, acyl, alkoxycarbonyl, halo-substituted alkoxycarbonyl, unsubstituted or substituted allyloxycarbonyl, etc.;

hydroxy protecting group - represents a group which will protect the hydroxy group during a desired chemical reaction such as the reactions described above, e.g., suitable hydroxy protecting groups include benzyl, substituted benzyl, alkoxy-substituted phenyl, acyl, alkoxycarbonyl, halo-substituted alkoxycarbonyl, or unsubstituted or substituted allyloxycarbonyl,

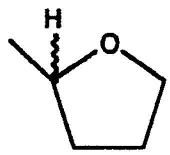 , 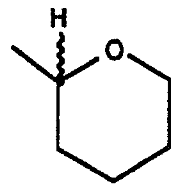 , 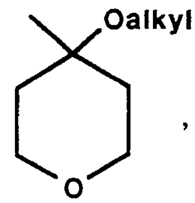 ,

alkyl, alkyloxyalkyl, alkylthioalkyl or $R'R''R'''$silyl where $R'$, $R''$ and $R'''$ may be the same or different and each is selected from alkyl, cycloalkyl or phenyl; and

substituted allyloxycarbonyl - represents an allyloxycarbonyl group in which the allyl group is substituted with halo, phenyl, substituted phenyl, etc.

substituted phenyl, substituted benzyl, substituted benzoyl, substituted 3-thienyl and substituted 3-furanyl - represent groups in which the phenyl, thienyl or furanyl rings thereof are substituted with one or more subsituent groups each independently selected from halo, alkyl, hydroxyalkyl, hydroxy, alkoxy, alkylthio, phenoxy, alkanoyl, benzoyl, alkanoyloxy, benzoyloxy, amino, alkylamino, dialkylamino, etc.

The novel processes and intermediates of the invention are illustrated in the following schematic reaction sequence, wherein Ar, $Ar^1$ R, $R^1$, $R^2$, P, X and $X^1$ are as defined above:

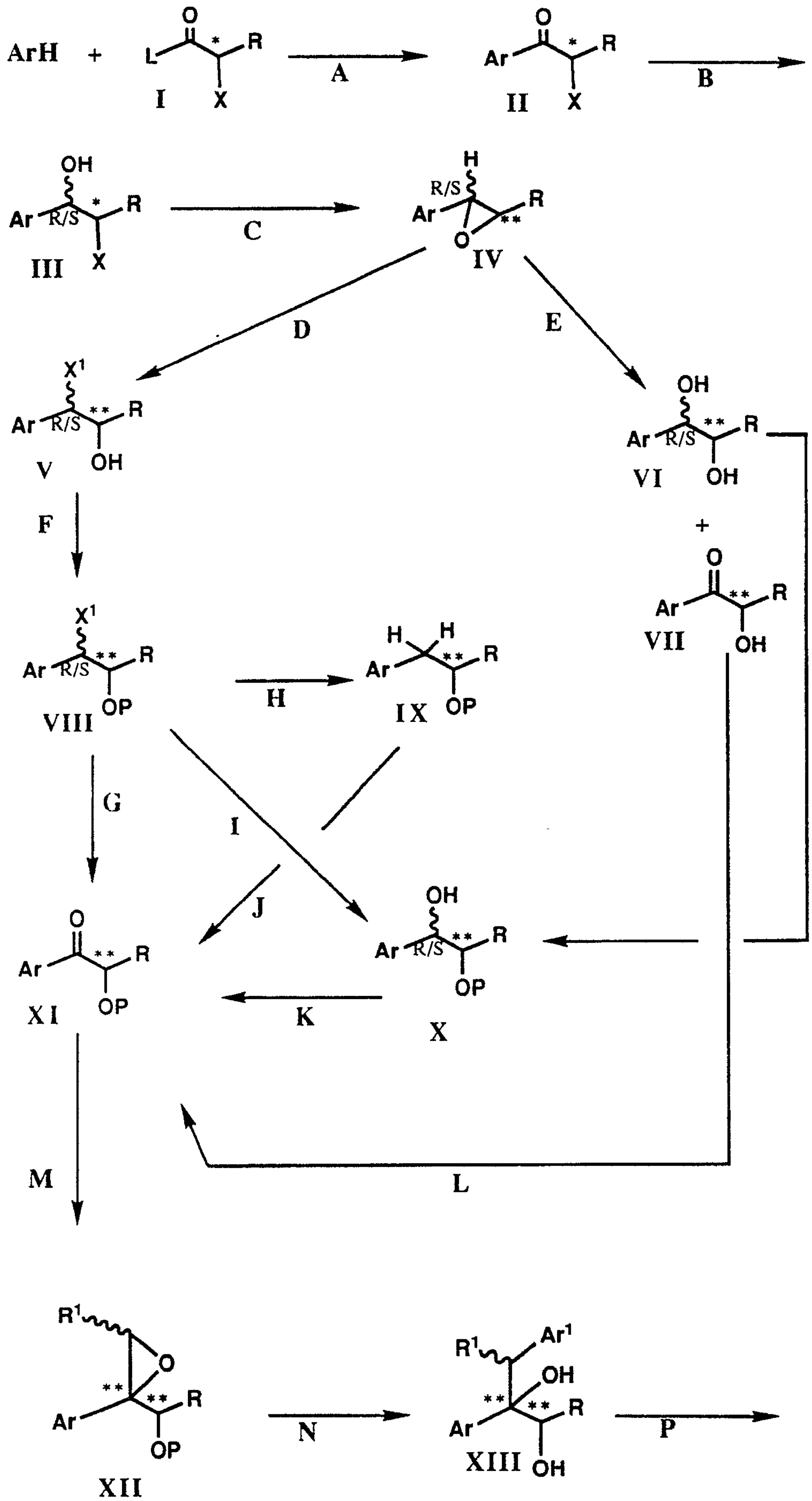


The asymmetric carbon center indicated by the single asterisk (*) in formula I is preferably in its S

absolute stereochemical form and will thus result in an end product of formula XVI having R absolute stereochemical configuration at both of the double asterisked (**) carbon centers, i.e., the RR form of the compound of formula XVI. If the compound of formula I has the R absolute stereochemical configuration at the single asterisked (*) carbon, both of the carbon atoms indicated by the double asterisked (**) in the final product XVI will be in their S absolute stereochemical configuration, i.e., the SS form of the compound of formula XVI.

In Step A, L can be any suitable leaving group such as a halo, acid anhydride or OH, preferably halo such as chloro. The reaction mixture may include a suitable solvent such as a nonreactive, nonpolar solvent, e.g., the reactant ArH itself, $CH_2Cl_2$, metadichlorobenzene, $CS_2$, etc. Any suitable temperature may be employed. Preferably, the reaction mixture is cooled, e.g., to about 0° C, and a suitable Lewis acid is added. Exemplary Lewis acids include $AlCl_3$, $SnCl_4$, $FeCl_3$, etc. The Lewis acid may be present in amounts from about 1 equivalents to about 3 equivalents, preferably in about equivalent amounts. The reaction may be quenched by pouring into water, preferably ice water and the product extracted with a suitable solvent.

In Step B, the ketohalide of formula II is reacted with a suitable non-basic, reducing agent such as $NaBH_3CN$, borane, alkyl boranes, etc. Preferably, the reaction is run in a suitable solvent such as methanol, tetrahydrofuran, diethylether, etc. The reducing agent is preferably employed in excess, e.g., in about a 0.5 to about 1.0 molar excess. The reaction may be run at any suitable temperature, e.g., room temperature.

In Step C, the halohydrin of formula III is reacted with a suitable base or acid scavenger such as $K_2CO_3$, OH-, organic base, etc. sThe reaction is preferably performed in a suitable polar solvent such as methanol, ethanol, dimethyl sulfoxide (DMSO), etc. The reaction may be run at any suitable temperature, e.g., from about 0 to about 80° C. The base is preferably employed in amounts of from about 1 to about 1.5 molar amounts, preferably in about equal molar amounts. Typically, the solvent is removed and the residue washed with water and extracted in $CH_2Cl_2$. At this point, it should be noted that the stereochemistry at the single asterisked carbon (*) in compound of formula III has been inverted at the double asterisked (**) carbon in the compound of formula IV.

In Step D, the epoxide of formula IV can be selectively opened at the aryl substituted carbon by using any suitable epoxide opening reagent such as halotrialkylsilane, phenoxide, substituted phenoxides, aniline, substituted anilines, benzyl alcohol, substituted benzyl alcohols, acylamines, alkoxycarbonylamino, alkyl or aryl mercaptan, etc. The reaction may be run neat or in a suitable solvent, e.g., with halotrialkylsilane, dry solvent such as $CH_2Cl_2$, or with an alkoxy substituted sodium phenoxy salt in methanol, etc. Any suitable temperature may be employed, preferably low temperature below about -20° C to about 80° C. The ring opening agent is typically added slowly to the epoxide solution in amounts of from about 1.0 equivalent to about 5.0 equivalents. The reaction is neutralized and the product extracted in a suitable solvent such as methylene chloride.

In Step F, the compound of formula V is reacted with a suitable protecting agent such as dihydrofuranyl ether, dihydropyranyl ether, alkoxyalkyl or alkylthioalkyl ether unsaturated or halo derivatives (such as a vinyl alkyl ether or a chloro-alkyl alkyl ether), halotrisubstituted silyl ethers, etc. A preferred protecting agent is for example dihydropyranyl ether. Typically, the compound of formula V is reacted with an excess of the protecting agent. A catalyst such as a Lewis acid may be employed. A preferred catalyst is $POCl_3$. The reaction may be run at any suitable temperature, e.g., 0° C to room temperature. Typically, the reaction mixture is then washed with base such as aqueous sodium carbonate and extracted with a suitable solvent.

In Step G, the protected compound of formula VIII is reacted with a suitable oxidizing agent such as DMSO if $X^1$ is halo. In other situations, the $X^1$ group is first converted to the hydroxide and then to the keto group. For example, ceric ammonium nitrate can be employed if $X^1$ is for example alkoxy phenoxy or dialkylaminophenoxy, and the resulting hydroxy group can be oxidized by a conventional oxidizing agent such as manganese dioxide, Jones reagent, chromium trioxide, etc. If $X^1$ is an alkyl or aryl mercapto group, an oxidizing agent such as chlorine or sulfuryl chloride may be employed, which after hydrolysis results in the ketone of formula XI. If $X^1$ is an acyloxy group, hydrolysis with a suitable hydrolizing agent such as aqueous sodium hydroxide can be performed and the resulting hydroxy group oxidized to a keto group using a conventional oxidizing agent as explained above.

Ketone XI can also be prepared from compound IV by following Step E. Here the epoxide IV is reacted with a dialkyl sulfoxide such as dimethyl sulfoxide in presence of a Lewis acid such as boron trifluoride, trimethyl silyl triflate, metal Lewis acids like $Zn^{++}$, $Ti^{4+}$, $Zr^{4+}$, $Sn^{4+}$, $Mg^{2+}$, etc. The reaction may be run either neat, in DMSO or in a nonparticipating solvent like $CH_2Cl_2$ at temperatures ranging from about -20° C to about 60° C, preferably at about 10° C to about 30° C. The ratio of the hydroxy ketone VII to the diol VI varies with the conditions and the Lewis acid. The desired product VII is then reacted with a hydroxy protecting agent (step L) as discussed above in presence of nonenolizing acid catalyst. Note that the chirality alpha to the keto group remains intact during the hydroxyl protection step.

The by-product VI formed in the reaction step E when a hydrolyzing agent such as water is present can also be converted to the ketone XI by first selectively protecting the hydroxy group (Step U) using a suitable hydroxy protecting agent as discussed above to give the compound of formula X, where R is attached to the carbon bearing the group OP. The remaining unprotected hydroxyl group where Ar is attached is then oxidized to a ketone in step K by conventional agents, such as $MnO_2$, $CrO_3$, Jones reagent, $KMnO_4$ etc.

The keto compound of formula XI can also be prepared sfrom compound IX (Step J) by oxidation with reagents such as $KMnO_4$, selenium dioxide, $CrO_3$, $K_2Cr_2O_7$, air in presence of base

$$(O_2 + \overset{\ominus}{O}H),$$

etc., in a nonparticipating solvent such as acetone, methylene chloride (two phases) acetonitrile, DMF, etc., preferably in acetone at temperatures ranging from about 0 °C to about 30 °C. Compound IX may be prepared from compound VIII in step H, where the $X^1$ group is halo, OH or a hydroxy derived leaving group such as acyloxy or mesylate, by reduction to a methylene group by reagents such as $H_2$, trialkyl tin or hydride, zinc, tin, sodium, or magnesium metals, etc. in an appropriate solvent as known in the art.

In Step M the keto compound XI is reacted with the anion of a dialkyl sulfoxide of the formula

$$R^1\overset{\ominus}{C}H\text{-}S(O)CH_3$$

where $R^1$ is H or alkyl as defined. Typically, the reaction may be conducted in a nonparticipating polar solvent such as dimethyl formamide (DMF), dimethyl sulfoxide and the like. The reaction may be run at a suitable temperature from 0 °C to 50 °C, preferably at 0 °C to 10 °C. The anion of the sulfoxide may be prepared by employing strong bases such as sodium hydride, lithium or sodium hexa-alkyl disilazane, sodium or potassium amide, etc., neat or in the same eluant as the keto compound XII. When the reaction is complete, it is neutralized, e.g., to pH 7, and the epoxide XII is isolated by extraction with a suitable solvent such as methylene dichloride or ethyl acetate. At this point, it should be noted that the new chiral center formed at the carbon attached to the epoxy ring has the same relative stereochemistry as the carbon attached to the group OP. For example, if the stereochemistry of the ketone of formula XI is R where indicated by the double asterisk (**), the the newly formed center at the carbon attached to the epoxy group also has R stereochemistry and if ketone XI is S where indicated by the double asterisk (**), then the new product has S stereochemistry at the other the double asterisked (**) center. Thus, by starting with the appropriate R or S form of the compound of formula 1, the stereochemistry of the compounds of formulas XII, XIII, XIV, XV and XVI can be predetermined as desired.

In Step N the epoxide XII is reacted with an $Ar^1$ anion. The reaction favors regioselectivity at the center where $R^1$ is attached. When $Ar^1$ is a heterocycle like 1-(1,2,4 triazolyl), 1-(1,3,4-triazolyl) or imidazolyl, the anion is formed by reaction with a strong base such as sodium hydride, sodium or potassium amide, cesium hydroxide etc. The choice of the solvent is a polar nonparticipating solvent such as dimethyl formamide, N-methyl pyrrolidinone, hexamethyl phosphoramide (catalytic amounts), dimethyl sulfoxide, etc., at temperatures ranging from about 0 °C to about 120 °C, preferably in dimethyl formamide at 70 to 80 °C.

When the reaction is over, it is acidified in step N with a strong acid such as aqueous HCl, $H_2SO_4$, phosphoric or trifluoroacetic acids. This procedure enables cleavage of the oxygen protecting group generating the free hydroxyl group. Compound XIII is isolated by adjusting the pH of the reaction, e.g., to a pH of about 8 to 9, and extracting the product in a suitable solvent. Alternatively, product XIII can be precipitated out of the medium during adjusting the pH to a value of 8 to 9.

Steps P, Q and W may be performed as described in European published application No. 0178533. For example, the compound of formula XIII is reacted with a methane sulfonating agent such as methane sulfonyl chloride in an inert solvent (e.g., benzene, dichloromethane, etc.) in the presence of a base at about 0 °C. to about 30 °C to give the methane sulfonate of the formula XIV. The sulfonating agent is typically employed in excess (1 to 2 equivalents). The base can be, for example, an organic amine, e.g., pyridine.

The methane sulfonate of formula XIV is then reacted with a mercaptan anion of the formula $R^2S$- where $R^2$ is alkyl, cycloalkyl or cycloalkylalkyl. The counterion is preferably an alkali metal salt and the solvent is preferably inert (e.g., DMSO, DMF, etc.). The thiol salt may be employed in excess (2 to 10 equivalents) and the temperature is typically 0 to 80 °C.

The sulfide of the formula XV is then oxidized in step W using an oxidizing agent such as a peracid,

e.g., meta-chloroperbenzoic acid, in an inert solvent, e.g., chloroform, at a temperature of from about -30C to reflux.

The following examples are intending to illustrate, but not to limit, the present invention

## EXAMPLE 1

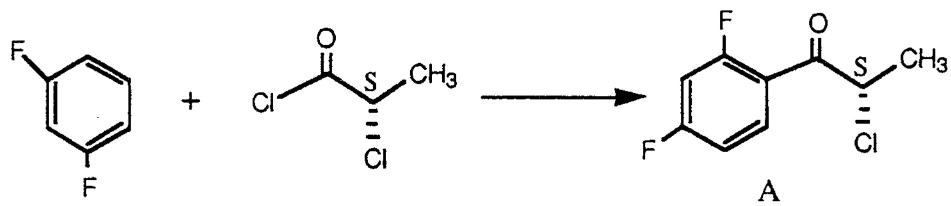

A

Add 1 g of S-(-)-2-chloropropionic acid, 1.5 g of mdifluorobenzene, .035 ml of dimethylformamide (DMF) to a flask and cool to 0° C. Slowly add 1.28 g of oxalyl chloride and stir for about 16 hrs. Add the resulting reaction mixture to 1.25 g of $AlCl_3$ in 1.05 g of mdifluorobenzene. Stir for about 6 hrs. at 0° C add 50 ml methylene chloride and pour into ice water. Let stand 1 hour. Separate $CH_2Cl_2$ layer and wash with $H_2O$, brine. Dry over $Na_2SO_4$. Remove the solvent by evaporation on a rotovap to yield 1.78 grams of the product of the formula A above.

## EXAMPLE 2

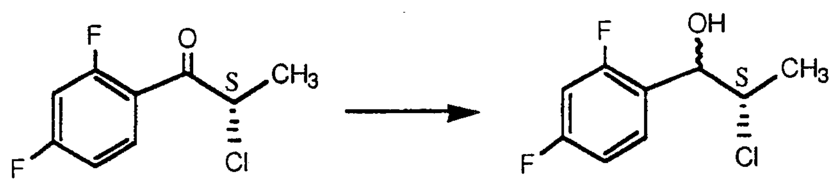

A B

Add 1.5 g of the product A of Example 1 above, 15 ml of THF 1.5 ml of acetic acid and add thereto 1.83 g of $NaBH_3CN$ and let stir overnight. Remove the solvents on a rotovap and add 25 ml of methylene chloride and 10 ml of 75% saturated aqueous brine solution and stir for about 25 minutes. Separate the methylene chloride and wash twice with 75% saturated brine. Dry the methylene chloride solution over $Na_2SO_4$. Remove the solvents by rotovap to provide 1.53 g of the desired product of formula B above.

## EXAMPLE 3

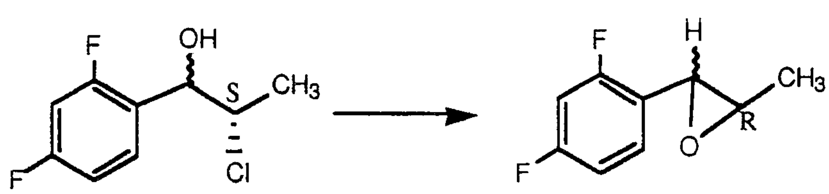

B C

Add 15.1 g of the product of formula B from Example 2 above, 150 ml of methyl alcohol (distilled over $CaH_2$), and 10.1 g of powdered $K_2CO_3$ and let stir at room temperature until TLC indicates disappearance

of starting material. Filter off the $KHCO_3$ and remove the methyl alcohol under vacuum at about 40 °C on a rotovap. Add methylene chloride to the residue and wash twice with water and then brine and dry over $Na_2SO_4$. Distill off the methylene chloride on a rotovap to provide 11.28 g of the product of formula C above as an oil.

## EXAMPLE 4

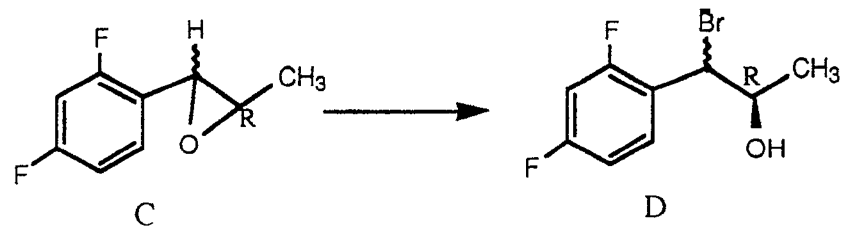

Add 2 g of the compound of formula C above from Example 3, 10 ml of methylene chloride and cool this mixture with an ice/acetone bath. Add 1.7 ml of bromotrimethyl silane slowly over 10 minutes and then let stir for about 45 minutes while warming to 0 °C. Stir the resulting reaction mixture with 50% saturated brine solution, separate wash with saturated brine and dry over $Na_2SO_4$. Remove the solvents by rotovap to provide 2.79 g of a group product of formula D above.

## EXAMPLE 5

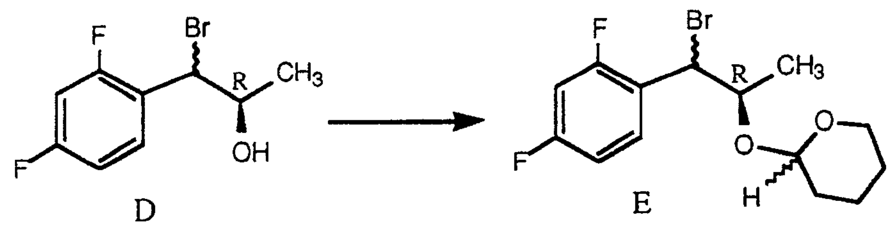

Add 0.5 g of compound of formula D above from Example 4, 10 ml of dry $CH_2Cl_2$, 1.81 ml of dihydropyran and 15 mg of $POCl_3$ to a flask and stir at room temperature for 4 hrs. Add saturated sodium bicarbonate solution and stir for 5 minutes, separate, wash with water and dry over $Na_2SO_4$. Purify the resulting product by running it over a course silica column eluting with a gradient of 2.5% $CH_2Cl_2$/hexane AE 30% $CH_2Cl_2$/hexane to provide 498 mg of the desired product of formula E above.

## EXAMPLE 6

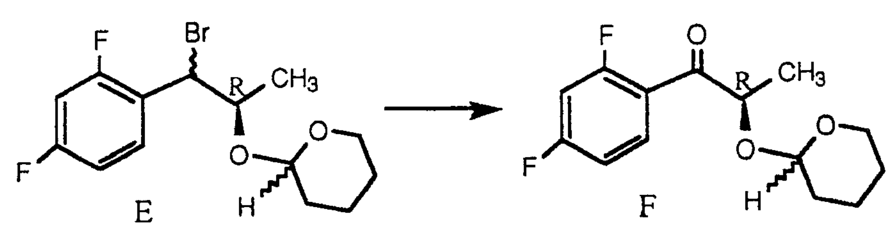

Add 1 g of the compound of formula E from Example 5 above, 5 ml of dry dimethylsulfoxide and 752 mg of silver benzoate to a flask. Heat the reaction mixture to 60 °C for 2 hrs. and cool to room temperature.

Add 50 ml of ether and wash with water containing 275 mg of sodium bicarbonate, water and then brine and dry over $Na_2SO_4$. Remove the solvent by rotovap to provide a light yellow oil and run the product over a course silica gel column with a gradient of 10% AE 50% $CH_2Cl_2$/hexane as eluant to provide 350 mg of the desired product of formula F above.

## EXAMPLE 7

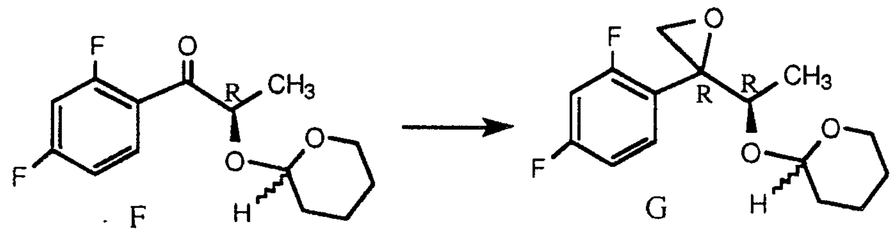

F G

Add 36 mg 50% NaH in oil to a vial and washed twice with 0.5 ml of hexane. Decant the hexane and dry with a $N_2$ flow. Add 0.5 ml of dry DMSO and stir for 5 minutes. Add 163 mg of $(CH_3)_3S(O)I$ and stir for about 2 hrs. Add 100 mg of a compound of formula F from Example 6 above and 0.5 ml of dry THF and stir for 3 about hrs. Pour in 5 ml of ice water. Extract the reaction mixture with diethyl ether. Wash the ether with water and brine and dry over $Na_2SO_4$. Filter and remove the solvent by rotovap to give 88 mg of the product of formula G above.

## EXAMPLE 8

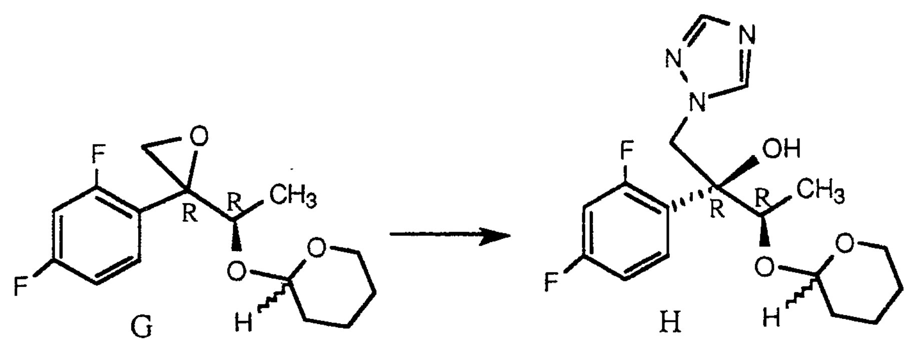

G H

Add 85 mg of sodium triazole and 0.5 ml of dry DMF to a flask and stir for 30 minutes. Add 88 mg of a compound of formula G from Example 7 above in 0.5 ml of DMF and let stir for 4 hrs. at 90° C. Cool the reaction mixture to room temperature, add toluene and wash with water and then brine and dry over $Na_2SO_4$ to give 82 mg of a compound of formula H above.

## EXAMPLE 9

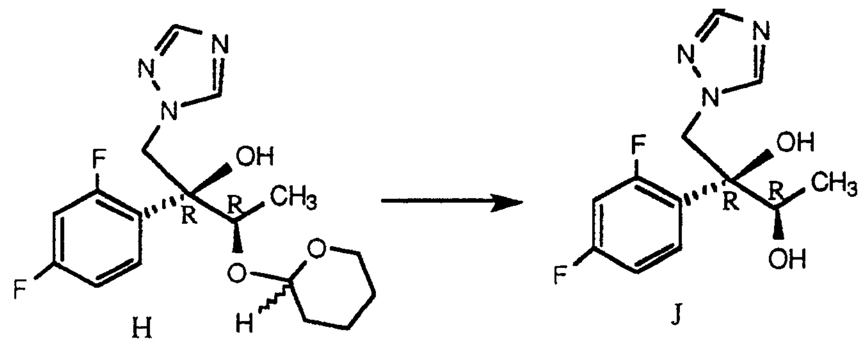

H J

Add 82 mg of a compound of formula H from Example 8 above to 0.2 ml of 4N HCl under a nitrogen blanket and let stir at room temperature for about 4 hrs. Work up by adding dropwise 25% aqueous sodium hydroxide until pH 9 and precipitate forms. Add about 5 ml of methylene chloride. Dry the methylene chloride solution with sodium sulfate anhydrous to remove water, then decant the methylene chloride solution and extract the residue twice with methylene chloride. Dry the methylene chloride solution over magnesium sulfate and concentrate to dryness on a rotovap to give 81 mg of a crude product. Purify the product by chromatography on a silica gel column with a gradient of first methylene chloride going to 1% methanol/methylene chloride to 2% and then 3% of methanol/methylene chloride to yield 22.9 mg of a compound of formula J above.

$[\alpha]_D^{26}$ = -70 (1% methanol). FAB Mass Spec. ($M^+$ + H) = 270.

## EXAMPLE 10

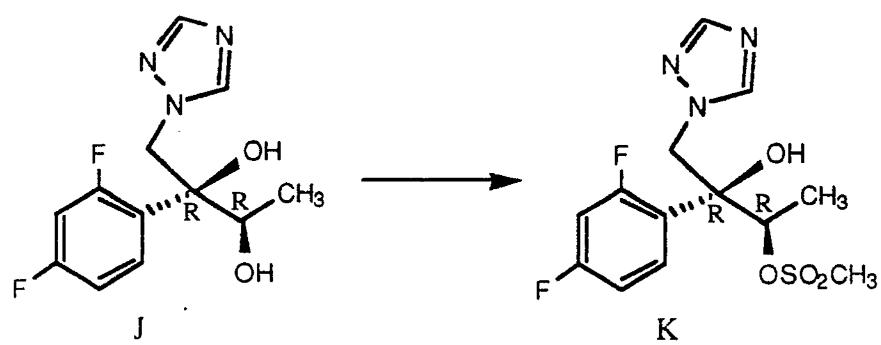

J K

Add 259 mg of the compound of formula J above, 2.5 ml of dry methylene chloride, and 0.4 ml of triethylamine. Cool the resulting solution to 5° C and add dropwise 0.1 ml of mesylchloride. Remove the cooling bath and stir at room temperature until the reaction is complete as indicated by TLC. Dilute the reaction mixture with methylene chloride, wash twice with water, and dry over sodium sulfate. Remove the solvent by rotovap to yield 372 mg of the mesylated compound of formula K above.

## EXAMPLE 11

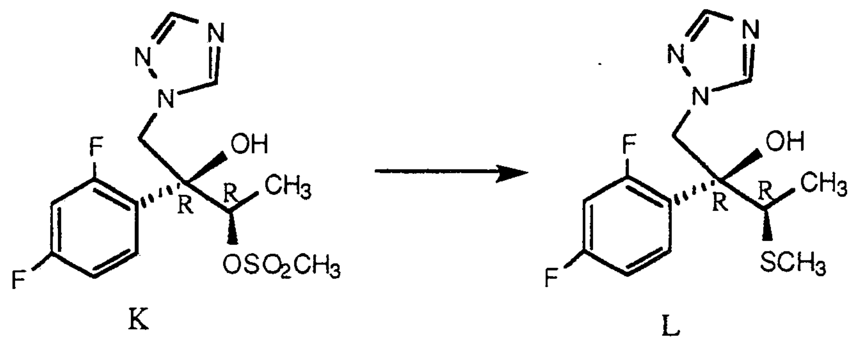

Add 372 mg of the mesylate of formula K above to 1.3 ml dimethylsulfoxide. Add 1.4 ml of 15% aqueous sodium methyl sulfide at room temperature. The temperature rises to about 45° C. Warm the reaction solution to 55° C and stir at this temperature for 2 hours. The product precipitates out of solution. When the reaction is complete as indicated by TLC, add 3.3 ml of ice water. Let the reaction mixture stir at -5° C for 15 minutes. Filter off the product, wash with ice water, then once with hexane. Dry the product in a vacuum oven at 40° C to yield 232 mg of the compound of formula L above.

## EXAMPLE 12

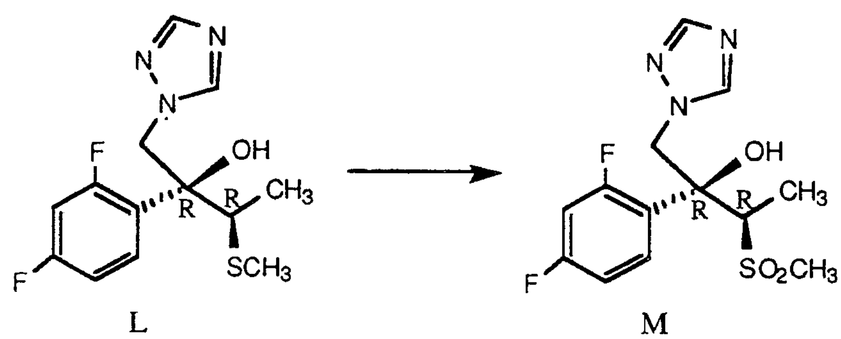

Dissolve 220 mg of the compound of formula L above in 1.3 ml chloroform. Cool the solution to 5° C and add portionwise 299 mg of meta-chloro-perbenzoic acid (85%). Remove the cooling bath and let the reaction mixture stir at room temperature for 2 hours. When TLC indicates the reaction is complete, the insolubles are filtered off. Wash the precipitate once with methylene chloride. The filtrate is washed with 10% sodium carbonate solution and then with brine. Dry the solution over sodium sulfate, and evaporate to dryness on a rotovap. Triturate the residue with ethyl ether to produce a white crystalline solid which is filtered to yield 223 mg crude product. Crystallize the crude product in acetonitrile to yield 117 mg of the compound of formula M above.

While the present invention has been described in connection with the foregoing examples, many variations and alternatives are possible. All such variations and alternatives are intended to be within the scope of the following claims.

## Claims

1. A compound of the structural formula II, III, IV, VIII, XI, or XII, wherein said compound has an asymmetric center(s) as indicated by the single asterisk (*) and the double asterisk (**) and wherein the compound is substantially free from compound(s) having the same chemical structure but having other absolute stereochemical configurations at the asterisked carbon centers:

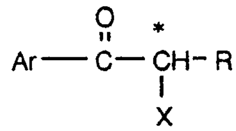

II,

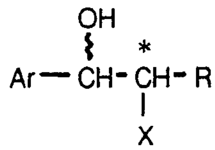

III,

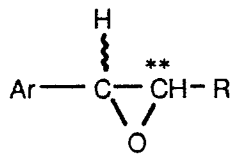

IV,

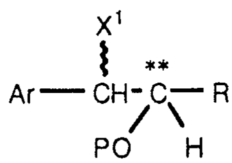

VIII,

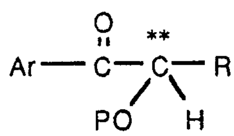

XI or

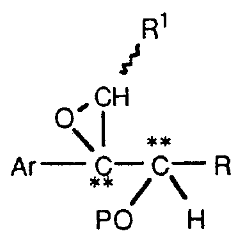

XII

or a pharmaceutically acceptable salt thereof,
wherein Ar represents phenyl, substituted phenyl, 3-thienyl, substituted 3- thienyl, 3-furanyl or substituted 3-furanyl; R represents alkyl or cycloalkyl; $R^1$ represents H or alkyl; X represents chloro, bromo or iodo; $X^1$ represents OH, chloro, bromo, iodo or $P^1$ where $P^1$ represents alkoxy-substituted phenyloxy, benzyloxy, substituted benzyloxy, acyloxy, alkylthio, phenylthio, substituted phenylthio or $-NHP^2$ where $P^2$ is an amino protecting group; and P represents H or a hydroxy protecting group.

2. A compound according to claim 1, wherein the carbon atoms indicated by the single asterisk (*) are in their S absolute stereochemical configurations and the carbon atoms indicated by the double asterisk (**) are in their R absolute stereochemical configurations.

3. A compound according to claim 1 having the formula XII.

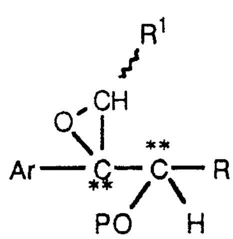

XII

wherein Ar, P, R and $R^1$ are as defined in claim 1.

4. A compound according to claim 1,2 or 3, wherein Ar is 2,4-difluorophenyl, wherein R is methyl, wherein P is 2-tetrahydropyranyl, wherein $R^1$ is H and wherein X is chloro or bromo.

5. A process for preparing a compound of formula IV set forth in claim 1 comprising reacting a compound of the formula ArH wherein Ar represents phenyl, substituted phenyl, 3-thienyl, substituted 3-thienyl, 3-furanyl or substituted 3-furanyl, with a substantially pure, enantiomeric compound of formula I

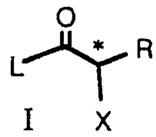

I

wherein the the asterisk (*) indicates an asymmetric center and wherein R represents alkyl or cycloalkyl, L represents a leaving group, and X represents chloro, bromo or iodo, in the presence of a Lewis acid to form a compound of formula II

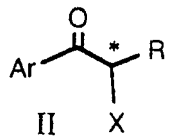

II

reacting the compound of formula II with a non-basic, reducing agent to form a compound of the formula III

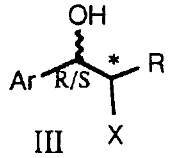

III

and reacting the compound of the formula III with a base to form a compound of the formula IV

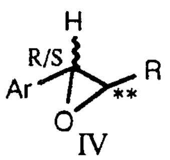

IV

wherein the asymmetric center in the compound of formula IV indicated by the double asterisk (**) is inverted with respect to the asymmetric centers indicated in compounds of formulas I-III above by the single asterisk (*).

6. A process for preparing a compound of formula V set forth in claim 1 comprising reacting a compound of the formula IV

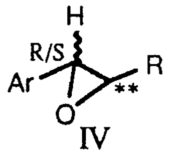

IV

wherein the the double asterisk (**) indicates an asymmetric center and wherein Ar represents phenyl, substituted phenyl, 3-thienyl, substituted 3-thienyl, 3-furanyl or substituted 3-furanyl, and R represents alkyl or cycloalkyl, with an epoxide opening agent to form a compound of the formula V

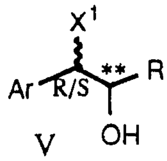

V

wherein X' represents OH, chloro, bromo, iodo or $P^1$ where $P^1$ represents alkoxy, phenyloxy, substituted phenyloxy, benzyloxy, substituted benzyloxy, acyloxy, alkylthio, phenylthio, substituted phenylthio or $-NHP^2$ where $P^2$ is an amino protecting group, and wherein the absolute stereochemistry at the the double asterisked (**) asymmetric center is substantially retained.

7. A process according to claim 6 for preparing compound of formula XI from a compound of formula V which comprises

a) reacting the compound of formula V with a hydroxy protecting agent to form a compound of the formula VIII

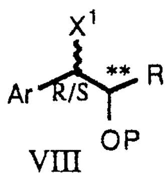

VIII

wherein P represents H or a hydroxy protecting group and Ar, $X^1$ and R are as defined in claim 6;

b)(1) reacting the compound of the formula VIII with an oxidizing agent to form a compound of the formula XI

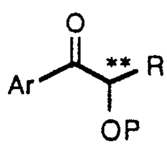

XI.

or b(2) or reacting the compound of the formula VIII with a reducing agent to form a compound of the formula IX

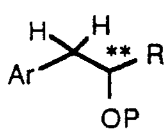

IX.

and thereafter reacting the compound of the formula IX with an oxidizing agent to form a compound of the formula XI

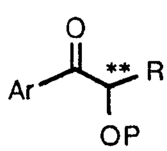

XI.

or b(3) reacting the compound of the formula VIII with an oxidizing agent to form a compound of the formula X

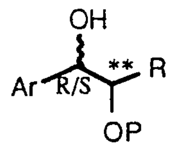

X.

and thereafter reacting the compound of the formula X with an oxidizing agent to form a compound of the formula XI

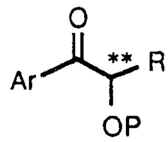

XI.

8. A process for preparing a compound of formula XIII comprising a reacting a compound of the formula IV

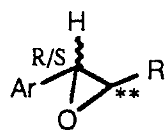

IV

wherein the the double asterisk (**) indicates an asymmetric center and wherein Ar represents phenyl, substituted phenyl, 3-thienyl, substituted 3- thienyl, 3-furanyl or substituted 3-furanyl, and R represents alkyl or cycloalkyl, with an oxidizing agent to form a compound of the formula VI and/or VII

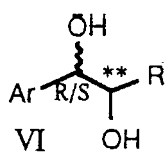

VI

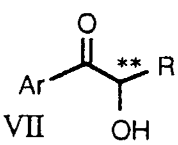

VII

wherein the absolute stereochemical configuration at the the double asterisk (**) asymmetric center with compounds of formula VI and/or VII is substantially retained with respect to that of the compound of formula IV;

b(1) reacting the compound of the formula VI with a hydroxy protecting agent to form a compound of the formula X

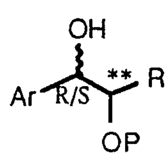

X.

or b(2) reacting the compound of the formula VII with a hydroxy protecting agent to form a compound of the formula XI

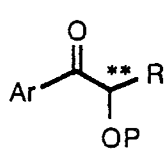

XI.

(c) reacting the compound of the formula XI with an epoxidizing agent to form a compound of the formula XII

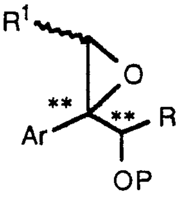

XII

wherein $R^1$ represents H or alkyl, and

(d) reacting the compound of the formula XII with a nucleophilic $Ar^1$ anion to form a compound of the formula XIII

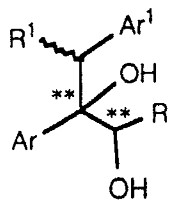

XIII,

wherein $Ar^1$ represents 1-(1, 2, 4-triazolyl, 1 -(1, 3, 4-triazolyl) or 1-imidazolyl.

9. A process for preparing a compound of the formula XVI

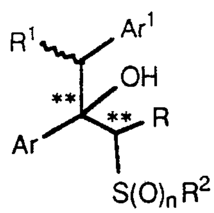

XVI,

which comprises applying methods known per se to a compound of the formula XIII

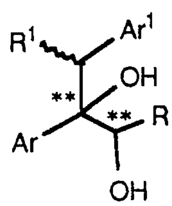

XIII,

wherein the asymmetric carbon centers are indicated by the double asterisks (**), wherein both the asterisk carbon centers (**) have the same absolute stereochemical configuration, and wherein said compound is substantially free from compounds having other absolute stereochemical configurations at such carbon centers:

wherein Ar represents phenyl, substituted phenyl, 2- or 3-thienyl, substituted 2- or 3-thienyl, 2- or 3-furanyl, substituted 2- or 3-furanyl, 2-,4- or 5-imidazolyl, 3- or 5-(1,2,4-triazolyl), 5-tetrazolyl, 2-,4- or 5-thiazolyl, 2-,4- or 5-oxazolyl, 3-,4- or 5-isoxazolyl, 2-,3- or 4-pyridinyl, or 2-,4- or 6- purinyl, wherein the saturated nitrogen atoms and said imidazolyl, triazolyl, tetrazolyl, benzimidazolyl, benzthiazolyl, and purinyl groups may be substituted with H, alkyl, substituted alkyl, cycloalkyl, substituted cycloalkyl, phenyl or substituted phenyl;

$Ar^1$ represents 1-(1,2 4-triazolyl), 1-(1,3,4-triazolyl) or 1-imidazolyl;

R represents alkyl, substituted alkyl or cycloalkyl;

$R^1$ represents H or alkyl;

$R^2$ is alkyl, cycloalkyl or cycloalkylalkyl; and
wherein n is 1 or 2.

10. The process of claim 9 wherein the methods known per se of claim 9 comprise

(a) reacting the compound of formula XIII with a methane sulfonating agent to form a compound of the formula XIV

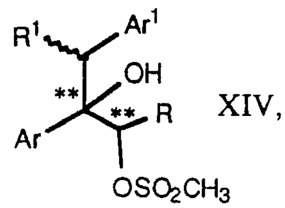 XIV,

(b) reacting the compound of formula XIV with a mercaptan anion of the formula $R^2S$ to form a compound of formula XV

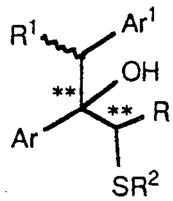 XV,

and (c) reacting the compound of formula XV with an oxidizing agent to form a compound of the formula XVI

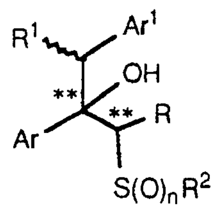 XVI,

wherein R, $R^1$, $R^2$, Ar, $Ar^1$ and n are as defined in claim 9.

European Patent Office

# PARTIAL EUROPEAN SEARCH REPORT

which under Rule 45 of the European Patent Convention shall be considered, for the purposes of subsequent proceedings, as the European search report

Application number

EP 90 30 9479

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 217 376 (ZAMBON S.p.A.)<br>* Claims *<br>-- | 1,2 | C 07 C 49/80<br>C 07 C 33/46<br>C 07 D 303/08<br>C 07 D 309/12<br>C 07 D 407/12<br>C 07 D 249/08<br>C 07 D 405/12 |
| X | EP-A-0 067 698 (SAGAMI)<br>* Claims *<br>-- | 1,2 | |
| Y | DE-B-1 270 022 (CHEMISCHE FABRIK KALK GmbH)<br>* Whole document *<br>-- | 1 | |
| Y | GB-a-1 340 033 (THE WELLCOME FOUNDATION LTD)<br>* Whole document *<br>-- ./. | 1 | |

**TECHNICAL FIELDS SEARCHED (Int. Cl.4)**

C 07 C 49/00
C 07 C 33/00
C 07 D 303/00
C 07 D 309/00
C 07 D 405/00
C 07 D 407/00
C 07 D 249/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 4

Claims searched incompletely: 1-3,5-10

Claims not searched:

Reason for the limitation of the search:

Article 84 and Rule 29 of E.P.C.: complicated claims formulation search limited on examples of the description.

| Place of search | Date of completion of the search | Examiner |
|---|---|---|
| THE HAGUE | 22-11-1990 | CHOULY |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# PARTIAL EUROPEAN SEARCH REPORT

Application number EP 90 30 9479



## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| Y | EP-A-0 278 346 (BAYER)<br>* Claims *<br>-- | 1-10 | |
| Y | EP-A-0 180 136 (BAYER)<br>* Claims *<br>-- | 1-10 | |
| Y | EP-A-0 158 448 (I.C.I.)<br>* Claims *<br>-- | 1-10 | TECHNICAL FIELDS SEARCHED (Int Cl 4) |
| D,Y | US-A-4 526 983 (SAJI et al.)<br>* Whole document *<br>-- | 1-10 | |
| D,Y | EP-A-0 178 533 (SUMITOMO)<br>* Claims *<br>---- | 1-10 | |